Europäisches Patentamt

European Patent Office

Office européen des brevets

(11) Publication number: **0 293 790**
**B1**

# EUROPEAN PATENT SPECIFICATION

(45) Date of publication of patent specification: **30.01.91**

(51) Int. Cl.⁵: **A 61 K 7/06**

(21) Application number: **88108562.5**

(22) Date of filing: **28.05.88**

(54) A composition for preventing loss of hair and facilitating growth of hair.

(30) Priority: **05.06.87 JP 140881/87**

(43) Date of publication of application:
**07.12.88 Bulletin 88/49**

(45) Publication of the grant of the patent:
**30.01.91 Bulletin 91/05**

(84) Designated Contracting States:
**DE FR GB IT**

(56) References cited:
**BE-A- 351 584**
**US-A-4 268 500**

**PATENT ABSTRACTS OF JAPAN, vol. 8, no. 236
(C-249)1673r, 30th October 1984; & JP-A-59 116
211 (POLA KASEI KOGYO K.K.) 05-07-84**

(73) Proprietor: **NIKKO SOGYO YUGEN KAISHA
No. 5-56-10, Asakusa Taito-ku
Tokyo (JP)**

(72) Inventor: **Matsuoka, Katsuo
No. 3-31-4-301 Senzoku
Taito-ku Tokyo (JP)**

(74) Representative: **Patentanwälte Dipl. Ing. R.
Splanemann Dr. B. Reitzner Dipl. Ing. K.
Baronetzky
Tal 13
D-8000 München 2 (DE)**

**Description**

The present invention relates to a composition for preventing loss of hair and facilitating growth of hair, in particular to a hair-treatment composition containing a characteristic natural plant component, and to a method for producing it.

Conventionally, a number of therapies and treatments were essentially carried out to prevent loss of hair and to facilitate growth of hair. Examples are the application of hair-growth facilitating agent, the physical stimulus or other on the skin having the bulb of hair to facilitate growth of hair, or some ornamental false hair in the form of wig or hairpiece.

The former is very preferable giving a natural hair, and the latter is preferable in a case of urgent, being instant when the natural hair is not obtained, being troublesome to wear and remove, sometimes falling off unexpectedly.

Consequently, although the natural hair is the most preferable, once it falls off, the recovery of it, that is, growth or treatment of hair is quite difficult.

Therefore, conventionally, in order to facilitate the natural growth of hair, various hair-growth facilitating agents, apparatuses or methods were provided, but these were not satisfactory.

Namely, as the conventional hair-growth or treatment agent, various ones such as ones comprising naturally occurring natural drug as major component (See, for example, the Japanese Patent Publication No. 58—11923 for one containing an extract from *Patasites japonica Maxim* or *Tussilago farfara L.*, the Japanese Patent Publication No. 58-40923 for one containing an extract from *Cirsium, Lycium chinese, Bupleurum falcatum, Cnidium officinale, H. nipponica* or other), or ones prepared by chemical methods (See the Japanese Patent Publications Nos. 59—7681, 59—14444, 60—34921 and 60—42204) were provided.

However, for the one comprising natural drug as a major component, it is fairly difficult to obtain the natural drug, for the one prepared by the chemical method, it is fairly difficult to manufacture it, and furthermore, the hair-growing effect has not been sufficiently satisfied.

*Illicium religiosum* Sieb. et Zucc. is an evergreen small tree belonging to the genus of Magnoliaceae, and is abundant everywhere to be easily available. Generally, it is known that higher plants produce various secondary metabolites such as alkaloid, flavonoid, anthraquinone, isoprenoid, lignin or cumalin, and that among them there are many compounds which provide an effective physiological activity by itself or in combination with other components. The shikimic acid which is named after the Japanese name of *Illicium religiosum* "Shikimi" is an important compound of biological significance in the biosynthetic pathway of these various secondary metabolites, various polyphenols such as lignin, flavonid, cumalin or phenylpropanoid being produced through the secondary metabolite system coming from the shikimic acid pathway having the shikimic acid as an important metabolic intermediate. Aromatic amino acids such as tyrosine, phenylalanine or tryptophan, and aromatic compounds such as p-aminobenzoic acid as considered to be biosynthesized through the shikimic acid pathway. Of course, the plant *Illicium religiosum* has the shikimic acid pathway using the shikimic acid as the metalbolic intermediate, being considered to be a preferable material which is easily obtainable, strong in secondary metabolic capability and contains various plant secondary metabolites.

Therefore, we pursued the possibility of significant effectiveness of *Illicium religiosum* extract on hair-growth or treatment for human body, and tried to obtain a hair treating agent comprising it as major component.

Thus, an object of the present invention is to provide a composition for preventing loss of hair and facilitating good growth of hair, being effective and safe without any side effect.

Accordingly, the present invention provides a composition for preventing loss of hair and facilitating growth of hair characterized in that said composition contains an extract from *Illicium religiosum*.

*Illicium religiosum* is an evergreen small tree belonging to the genus of Magnoliaceae, and any roots, root skins, trunks, barks, leaves, flowers or fruits of which are available in the present invention, the hair-treatment composition of the present invention being made by extracting the effective component to prepare an extracted essence, followed by adding this extracted essence to the composition.

For the solvent and method for extraction, various conventionally known ones are applicable, for example, as the solvent for extraction water, methanol, ethanol or other is used, while as the method for extraction plant material is optionaly dried, mechanically ground before immersion in the solvent and followed by optional heating, agitating or ultrasonic treatment. After the extraction of the *Illicium religiosum* plant component, the extracted essence is prepared by concentrating the extract, and the extracted plant component has a strong affinity to solvents (especially to water) because it is a mixture comprising various components, not always being dried up completely by the usual concentration operation under vacuum depending on the preparation method, becoming a solid in case of drying up, or becoming a steady state in a fluid form during the concentration process retaining a certain amount of solvent in case of not drying up. Said "extracted essence" means the *Illicium religiosum* plant extract in an abovementioned state, the hair-treating composition of the present invention being made by adding this *Illicium religiosum* extracted essence in suitable concentration.

The extraction of the effective component may also be carried out by drying the root or other of *Illicium religiosum* to grind, treat by hot water and steam, and the resulting extract solution has a natural color of

EP 0 293 790 B1

orange. Alternatively, the extract solution may be treated to be powder.

The hair-treatment composition of the present invention can be used in any form of solution, cream or emulsion.

For example, hair-tonic, hair-lotion, hair-cream, shampoo or hair-rinse may be made in a desired form, for example, liquid, emulsion or paste, using for example an extract in a form of powder by the usual method. Wherein usual additives conventionally used for hair-treatment composition, for example, distilled water, alcohols, polyols may be added as bases, furthermore, resorcinol, hormones, vitamins, nicotinic acid derivative, sensitizing dye, amino acids, sterilizer, menthol or other perfume may be simultaneously included as pharmaceutically effective components.

Alternatively, the obtained extracted component as powder may be mixed with milk. Namely, the extracted powder can be mixed with milk to make a form of yogurt by adding lactic acid bacteria. Wherein, agitation may preferably be carried out at about 10—80C preferably at about 60C to make a yogurt. Then, it is separated to liquid and solid, and dehydration treatment is carried out by evaporating water. After dehydration treatment, it becomes a form of powder, and this powder can be made into suitable form by the usual method, as described above.

These mixing with milk or dehydration treatment give no change to the hair-treatment effect of the effective component extracted from *Illicium religiosum*.

Alternatively, hot water may be added to the powder after dehydration to make a liquid form by agitating for 30—60 minutes.

According to the hair-treatment composition of the present invention, this may be applied externally to the skin. Thereupon, capillary vessels in the skin is enlarged to facilitate the circulation of the blood, and such situation in which hairs are easy to grow takes place by stimulating the activity of the bulb of hair.

The biochemical mechanism itself by which the hair-treatment composition of the present invention reacts to prevent loss of hair and facilitate growth of hair is not obvious. However, it is postulated that because *Illicium religiosum* is a plant having the strong secondary metabolic ability as described above, the effective and safe effects of preventing loss of hair and facilitating growth of hair is provided by the homogeneously mixed extracted components.

Therefore, according to the hair-treatment composition of the present invention, the extract of *Illicium religiosum* enlarges capillary vessels in the skin to facilitate the circulation of the blood, make hair to be in such situation that it easily grows by stimulating the activity of the bulb of hair, prevents loss of hair and white hair, has a significant effect in hair-treatment and growth of hair safely without any side effects.

The present invention will be further illustrated by the following examples.

Preparation of Illicium religiosum extract

Using 1,000 g of all of the root, leaf and trunk without the seed in *Illicium religiosum*, after steaming for 15—20 minutes it is dried. It may be dried either by natural drying method under sun or by mechanically forced drying method, resulting in 360 g of material by the drying.

The 360 g of dried material is immersed in 5 l of water, agitated at not less than 100C for 1—2 hours, extracted the effective component as a supernatant of it, to make the *Illicium religiosum* extract solution after filtration, to remove the residue. The extract solution is concentrated at atmospheric pressure, dried and ground to prepare a form of powder, resulting in 36 g of extract powder.

10 g of the extract powder is dissolved in 30—50 cc of hot water, agitated completely with 1 l of milk which has a fat concentration of 3%, added with lactic acid bacteria (volume 130 cc and weight 150 g) before complete agitation. And it is left for 7—10 days in an enclosed vessel at 60C. During the leaving, it becomes a form of yogurt after some 2 days, separating the water part from the solid part after 7 days.

Thus, the water is removed by heating to concentrate or by using a vacuum pot to make powder, resulting in 150 g of powder extract. It becomes preferable for long-term preservation owing to such treatment to make powder.

Alternatively, in the separation state after the leaving, a form which can be directly used may be obtained from this state. Namely, in the separation state after the leaving, it is heated at not less than 100C to boil, added with 1,500 cc of ethanol and 1,500 cc of distilled water and agitated, adjusted to be 4,000 cc as the total amount. And then, it is left until the supernatant becomes transparent, the supernatant being used optionally after filtration. The supernatant thus obtained is hair-tonic like, being able to use in the same way as hair-tonic, the obtained supernatant having a natural color of orange.

When the extract powder is dissolved in hot water, an amount of 30—50 cc, 50—100 cc or 100—150 cc of hot water is suitable for 10 g, 20 g, 30 g of extract powder, respectively.

Now the following result was obtained from the analysis for samples prepared by the extraction and preparation method described above using a gas-chromatograph (Hewlett-Packard, Co., Ltd., HP-5890J) and a gas-chromatograph-mass-spectrometer (JEOL, JMS—DX300).

Wherein this sample was prepared by dissolving 20 g of said extract powder in 50—100 cc of hot water, agitating completely with 1 l of milk which had a concentration of 3%, adding with lactic acid bacteria (volume 130 cc and weight 150 g), agitating completely, leaving for 7 days as described above, heating at not less than 100C to boil in the separation state after the leaving, adding with 1,500 ml of ethanol and 1,500 ml of distilled water, agitating, adjusting to be 4,000 cc as the total amount and then leaving until the supernatant becomes transparent to use the supernatant after filtration, 200 ml thereof being used.

3

Namely, the result of the analysis showed that water 63.2 (W/V%), ethanol 36.7 (W/V%), hexadecanoic acid 0.88 (mg/l), tetradecanoic acid 0.58 (mg/l), decanoic acid 0.46 (mg/l), dodecanoic acid 0.27 (mg/l), octadecanoic acid 0.23 (mg/l), C11 hydrocarbon 0.10 (mg/l), octanoic acid 0.10 (mg/l), and other organic compound (as n-dodecanoic acid) 1.61 (mg/l) were contained.

Preparation of hair-treatment compositions

The *Illicium religiosum* powder extract is added to the following components in a weight ratio of 10% to produce a hair-tonic as follows:

Composition of the components:

| | |
|---|---|
| Methanol | 10 parts by wt |
| Perfume | 0.2 parts by wt |
| 95% Ethanol | 45 parts by wt |
| Distilled water | 44.5 parts by wt |

Production method:

The above components are mixed before filtration.

The *Illicium religiosum* powder extract is added to the following components in a weight ratio of 10% to produce a hair-lotion as follows:

Composition of the components:

| | |
|---|---|
| Stearic acid (63) | 5 parts by wt |
| Cetanol | 0.5 parts by wt |
| Emulsifying agent | 0.8 parts by wt |

(Polyoxyethylene (20EO) sorbitan sesquioleate)

| | |
|---|---|
| Perfume | 1 part by wt |
| Triethanolamine | 0.4 parts by wt |
| Glycerol | 5 parts by wt |
| 95% Ethanol | 8 parts by wt |
| Distilled Water | 79.3 parts by wt |

Production method:

The above components are mixed before filtration.

The *Illicium religiosum* powder extract is added to the following components in a weight ratio of 10% to produce a hair-cream as follows:

Composition of the components

| | |
|---|---|
| Beeswax | 5 parts by wt |
| Anhydrous lanoline | 4 parts by wt |
| Vaseline | 5 parts by wt |
| Liquid paraffin | 33 parts by wt |
| Emulsifying agent | 4 parts by wt |

(Polyoxyethylene (20EO) sorbitan sesquioleate)

| | | |
|---|---|---|
| Perfume | 1 | part by wt |
| Borax | 1 | part by wt |
| Propyleneglycol | 5 | parts by wt |
| Distilled water | 42 | parts by wt |

Production method:
The above components were mixed.

The *Illicium religiosum* powder extract is added to the following components in a weight ratio of 5% to produce a shampoo as follows:
Composition of the components:

| | | |
|---|---|---|
| Sodium lauryl sulfate | 35 | parts by wt |
| Magnesium stearate | 4 | parts by wt |
| Polyvinyl alcohol | 1 | part by wt |
| Cetyl alcohol | 2 | parts by wt |
| Lanolin | 1 | part by wt |
| Glycerol laurate | 2 | parts by wt |
| Perfume | 1 | part by wt |
| Distilled water | 49 | parts by wt |

Production method:
The above components are mixed.

The *Illicium religiosum* powder extract is added to the following components in a weight ratio of 8% to produce a hair-rinse as follows:
Composition of the components:

| | | |
|---|---|---|
| Acetylated lanolin alcohol | 0.5 | parts by weight |
| Polyoxyethylene hardened castor oil | 0.5 | parts by weight |
| Perfume | 1 | part by weight |
| Distilled water | 90 | parts by weight |

Production method:
The above components are mixed.

Results of the tests of the hair-treating composition
The following results were obtained by applying the produced hair-tonic to subjects.
When it was applied to a subject (male of 60 years old) who had been in a proceeding of hair-loss state for more than 10 years, in the first place, the hair-loss and dandruff stopped within 10—30 days, secondly, hair-trunk appeared on the surface of the skin and it gradually became viable hair and hard hair. The application was 1—2 times a day, and the hair-trunk appeared at about one month after beginning of the application, and it became the hard hair of 2—3 cm after further 3—4 months. However, to this subject, the conventionally provided hair-treatment composition had no effect.
Alternatively, to a male subject of 56 years old who had suffered the loss of hair for more than 25 years, it was applied as described above, and the appearance of hair in the back of the head was found after one year. To a male subject of 53 years old who had been suffered the proceeding of loss hair for more than 30 years, it was applied as described above, and the appearance of hair all over the head was found after one year. To a male subject of 63 years old who had been in the state of loss of hair for 6—7 years, it was applied as described above, and the appearance of hair all over the head was found after one year. To a male

5

subject of thin hair of 44 years old, and to a male subject of 27 years old, it was applied as described above, and in both cases, the state of loss of hair stopped, and the hard hair appeared.

Furthermore, it was applied to a female subject of white hair of 77 years old as described above, the appearance of black hair was found within 2—3 months. To a female subject of 21 years who had essentially thin and slender hair it was applied as described below, and the hair became thick.

Any side effects such as skin disease or other were not found from these direct applications to the skin, and it was confirmed to be safe. Namely, also in the examination in the Chemicals Examination Association Foundation, any toxic substance such as R—Hg, T—Hg, Pb, Cd, Cr, Or—P, As, CN or PCB were not able to be found.

These hair-treating effects gave same effects upon ones whose hair losses were caused by any alopecia areata, alopecia atrophicans, alopecia diffusa or white hair.

## Claims

1. A composition for preventing loss of hair and facilitating growth of hair characterized in that said composition contains an extract from *Illicium religiosum*.

2. The composition according to claim 1, wherein said composition is in a form of solution.

3. The composition according to claim 1, wherein said composition is in a form of cream.

4. The composition according to claim 1, wherein said composition is in a form of emulsion.

5. The composition according to claim 1, wherein said composition contains one or more bases selected from a group consisting of water, alcohols and polyols.

6. The composition according to claim 1, wherein said composition contains one or more pharmaceutically effective components selected from a group consisting of resorcinol, hormones, vitamins, nicotinic acid derivatives, sensitizing dye, amino acids, sterilizer, menthol and perfume.

7. The composition according to claim 1, wherein said composition is in a form of yogurt prepared by mixing the extract from *Illicium religiosum* and a milk before adding lactic acid bacteria.

## Patentansprüche

1. Mittel zur Verhütung des Haarausfalls und zur Förderung des Haarwuchses, dadurch gekennzeichnet, daß es einen Extrakt aus *Illicium religiosum* enthält.

2. Mittel nach Anspruch 1 in Form einer Lösung.

3. Mittel nach Anspruch 1 in Form einer Creme.

4. Mittel nach Anspruch 1 in Form einer Emulsion.

5. Mittel nach Anspruch 1, enthaltend eine oder mehrere Grundlagen, ausgewählt aus der Gruppe Wasser, Alkohole und Polyole.

6. Mittel nach Anspruch 1, enthaltend eine oder mehrere pharmazeutisch wirksame Komponenten, ausgewählt aus der Gruppe Resorcin, Hormonen, Vitaminen, Nikotinsäurederivaten, Sensibilisierungsfarbstoffen, Aminosäuren, Sterilisatoren, Menthol und Parfum.

7. Mittel nach Anspruch 1 in Form eines Joghurts, der durch Vermischen des Extrakts aus *Illicium religiosum* und Milch vor der Zugabe von Milchsäurebakterien hergestellt wird.

## Revendications

1. Composition destinée à empêcher la perte des cheveux et facilitant la croissance des cheveux, caractérisée en ce que ladite composition contient n extrait de *Illicium religiosum*.

2. Composition selon la revendication 1, dans laquelle ladite composition se présente sous la forme d'une solution.

3. Composition selon la revendication 1, dans laquelle ladite composition se présente sous la forme d'une crème.

4. Composition selon la revendication 1, dans laquelle ladite composition se présente sous la forme d'une émulsion.

5. Composition selon la revendication 1, dans laquelle ladite composition contient une ou plusieurs bases choisies parmi un groupe incluant l'eau, des alcools et des polyols.

6. Composition selon la revendication 1, dans laquelle ladite composition contient un ou plusieurs composants efficaces du point de vue pharmaceutique, choisis dans un groupe incluant le résorcinol, des hormones, des vitamines, des dérivés de l'acide nicotique, un colorant de sensibilisation, des aminoacides, un agent de stérilisation, du menthol et du parfum.

7. Composition selon la revendication 1, dans laquelle ladite composition se présente sous la forme d'un yaourt préparé par mélange de l'extrait de *Illicium religiosum* et de lait avant l'addition d'une bactérie lactique.